# EUROPEAN PATENT SPECIFICATION

(11) **EP 4 114 443 B1**
(45) Date of publication and mention of the grant of the patent: **21.01.2026**
(21) Application number: 21710268.0
(22) Date of filing: 05.03.2021
(51) Int. Cl.: A61K 38/46, A61K 38/17, C07K 14/435, C12N 9/14, A61P 7/02, A61P 7/04

(54) **COMPOSITION COMPRISING APYRASE AND ANNEXIN THAT BINDS PHOSPHATIDYLSERINE FOR TREATMENT OF ARTERIAL AND VENOUS THROMBOSIS**
ZUSAMMENSETZUNGEN ENTHALTEND APYRASE UND ANNEXIN DASS PHOSPHATIDYLSERIN BINDET ZUR BEHANDLUNG VON ARTERIELLER UND VENÖSER THROMBOSE
COMPOSITION COMPRENANT DE L'APYRASE ET DE L'ANNEXINE QUI SE LIENT À LA PHOSPHATIDYLSERINE POUR LE TRAITEMENT DE LA THROMBOSE ARTÉRIELLE ET VEINEUSE

(30) Priority: 06.03.2020 US 202062986006 P
(43) Date of publication of application: 11.01.2023
(73) Proprietor: APT Therapeutics Inc., Naperville, IL 60540 (US)
(72) Inventor: CHEN, Ridong, Naperville, Illinois 60563 (US); JEONG, Soon, Seog, Naperville, Illinois 60565 (US)
(74) Representative: Latscha Schöllhorn Partner AG
(86) International application number: PCT/EP2021/055558
(87) International publication number: WO 2021/176038

(56) References cited:
- WO-A1-2005/099744
- WO-A1-2011/088231
- GB-A- 2 542 391
- US-A1- 2005 215 505
- US-B2- 8 771 683
- THIAGARAJAN PERUMAL ET AL: "Inhibition of arterial thrombosis by recombinant annexin V in a rabbit carotid artery injury model", CIRCULATION, AMERICAN HEART ASSOCIATION, US, vol. 96, no. 7, 1 January 1997 (1997-01-01), pages 2339 - 2347, XP002338645, ISSN: 0009-7322

## Description

### Technical Field

The invention is in the field of treatment of conditions characterized by unwanted thrombosis, such as heart attack, stroke, and complications of surgery where bleeding should be minimized. In particular it is directed to administering factors that bind phosphatidyl serine on the surface of activated platelets in competition with prothrombinase complex along with an apyrase for prevention or treatment of thrombosis.

### Background Art

The relevant conditions treated by the method of the present disclosure are both arterial and venous thrombosis.

In the case of arterial thrombosis, a high concentration of unfractionated heparin is routinely used to prevent peri-operative coagulation for patients undergoing coronary artery bypass surgery (CABG). Despite the use of protamine reversal, the heparin treatment increases post-operative bleeding and approximately 30 percent of patients require a blood transfusion after CABG. Other antithrombotic therapy also significantly increases the risk of postoperative bleeding. Clopidogrel (brand name: Plavix), prasugrel (brand name: Effient), ticagrelor (brand name: Brilinta), and ibuprofen are generally discontinued for several days prior to coronary surgery. Patients taking warfarin (brand name: Coumadin), apixaban (brand name: Eliquis), rivaroxaban (brand name: Xarelto), edoxaban (brand name: Savaysa), or dabigatran (brand name: Pradaxa) should stop taking it before surgery. As such, an antithrombotic treatment without causing peri or post-operative bleeding is critically needed.

Current adjunctive antithrombotic therapy during percutaneous coronary intervention (PCI), formerly known as angioplasty with a stent, also increases major bleeding. Coronary artery disease can be diagnosed by the presence of acute ST segment elevation myocardio infarction (STEMI) on an electrocardiogram. Platelets play a central role in thrombotic complication (Heit, JA, J Thromb Haemost (2005) 3:1611-1617). Treatment for this condition is directed to restoring normal coronary blood flow and to maximize salvage of the functional myocardium.

Current US and European guidelines recommend primary PCI along with adjunctive therapy which includes dual-antiplatelet agents plus an anticoagulant. However, the currently available agents with antiplatelet activity are delayed in the onset of action in about 40%-50% of STEMI patients (Riteau, N., et al., Am J Respir Crit Care Med (2010) 182:774-783) and the combinations all have mechanisms of action that increase bleeding. In a recent clinical trial, 11%-12% of patients suffered major bleeding (Ufer, M., Thromb Haemost (2010) 103:572-585). In addition, none of the current antithrombotic agents (*i.e.,* combinations of antiplatelet and anticoagulant agents) protect against reperfusion injury, which is defined as myocardial infarction associated with the restoration of coronary blood flow after ischemia. Reperfusion injury accounts for up to 50% of the final size of a myocardial infarct and causes related cardiac dysfunctions. This may explain why, despite optimal coronary reperfusion, that the rate of death after AMI approaches 10% and the incidence of heart failure is almost 25% (Heit, JA, J Thromb Haemost (2005) 3:1611-1617; Spyropoulos, AC, et al., J Manag Care Pharm (2007) 13:475-486; Marcus, AJ, et al., Semin Thromb Hemost (2005) 31:234-246).

Therefore, there is a long felt unmet medical need for fast acting therapeutic agents as adjuncts to PCI that will facilitate reperfusion without increasing bleeding and also attenuate reperfusion injury which will improve myocardial salvage and recovery of left ventricular function thereby reducing the incidence of heart failure.

A left ventricular assist device (LVAD) is a pump that is used for patients who have reached end-stage heart failure. The LVAD is a battery-operated, mechanical pump, and surgically implanted which then helps the left ventricle (main pumping chamber of the heart) pump blood to the rest of the body. LVADs can be used until a heart becomes available or patients can receive long-term treatment using an LVAD, which can prolong and improve patients' lives. Despite significant improvements in survival, functional capacity and quality of life, a common complication is pump thrombosis, stroke, and bleeding. As all the current antithrombotic drugs increase bleeding risk, achieving the optimal antithrombotic therapy that balances thrombosis prevention with increased bleeding has been an ongoing challenge.

For patients on chronic antithrombotic therapy who undergo surgery, current clinical recommendations suggest discontinuation of antithrombotic therapy prior to surgery as these drugs elevate risk for excessive bleeding. For example, stop aspirin around the time of surgery in patients who require CABG; stop unfractionated heparin 4-6h before surgery in patients, last dose of Low Molecular Weight Heparin 24h before surgery in patients. However, discontinuation of these antithrombotic treatments also increases the risk for peri-operative thrombosis such as pulmonary embolism, which can be highly fatal. Currently, there is no antithrombotic treatment that can be safely used for peri-operative management.

Similar disadvantages are found with regard to anticoagulant treatment for venous thromboembolism in that the treatment is plagued by increase major bleeding events and offers minimal protection against vein wall thickening or fibrosis. Venous thromboembolism including deep vein thrombosis (DVT) and pulmonary embolism (PE) is a major source of morbidity and mortality worldwide.

The current prophylaxis and treatment of DVT is administration of a parenteral anticoagulant, for example heparin, with subsequent transition to an orally active anticoagulant such as warfarin (Heit, JA, J Thromb Haemost (2005) 3:1611-1617; Kyrle, PA, et al., Lancet (2005) 365:1163-1174). Other agents have also been used-for example, Rivaroxaban has been approved for both short-term and long-term treatment (Perzbom, E., et al., Nature Rev. Drug Discovery (2011) 10:61-75; Ufer, M., Thromb Haemost (2010) 103:572-585), but all have mechanisms of action that produce bleeding which limits the dose that can be administered. In addition, the current treatments do not effectively protect against post-thrombotic syndrome (PTS) which is characterized by blood reflux caused by compromised vein valves, overflow obstruction and tissue hypoxia that is secondary to vein wall thickening and fibrosis (Popuri, RK, et al., Arterioscler Thromb Vasc Biol (2011) 31: 479-484; Saarinen, J., et al., J Cardiovasc Surg (2000) 41:441-446; Deatrick, KB, et al., J Vasc Surg 2011 53:139-146).

The impact of DVT is increasing with the growing aging population. In 2005, the United States Senate designated March as "Deep Vein Thrombosis Awareness Month." Clearly, the health and economic burden of DVT is profound and DVT patients would benefit greatly from an efficacious antithrombotic agent that is not limited by increased bleeding risk and also is capable of attenuating PTS.

As described below, the compositions and methods of the present invention offer a solution to this unmet medical need as well.

The use of apyrases, in particular soluble CD39L3 and modifications thereof, for treating conditions associated with thrombosis is disclosed in US 7,247,300; US 7,390,485 and US 8,021,866. Use of apyrases as therapy for bleeding conditions is disclosed in US 8,535,622 and an enhanced form of an apyrase based on soluble CD39L3 is disclosed in US 8,771,683. In addition, apyrase therapy for fibroproliferative disorders, pulmonary hypertension and heart failure is disclosed in US 14/666,121, currently pending.

A protein known to bind phosphatidyl serine on the surface of activated platelets in competition with the binding of prothrombinase complex, annexin V, is believed to be associated with a prevention and treatment of thrombosis in view of the showing that antibodies reactive with annexin V effect arterial and/or venous thrombosis, for example, in patients with systemic lupus erythematosus (Esposito, G., et al., Autoimmunity Rev (2005) 4:55-60). Indeed, although the primary use currently of annexin V is as label for apoptosis, it is understood that annexin V inhibits prothrombin activation and is able to prevent thrombus formation under normal venous and arterial blood flow conditions. Antibodies reactive with annexin V have been shown to result in thrombotic complications in patients with type 1 diabetes. (Bakar, F., et al., J Clin Endocrinol Metab (2014) 99:932-937).

It has now been found that annexin V or other annexins that bind phosphatidyl serine on the surface of activated platelets and an apyrase when administered in combination as such, or when administered as a single fusion protein, exert a synergistic antithrombotic effect and do not enhance unwanted bleeding.

Thus, this combination represents a solution to the problems currently associated with treatment of both arterial and venous thrombosis. Certain embodiments of annexin V coupled to an apyrase as a fusion protein have also been found to exhibit favorable properties with respect to recombinant production.

### Disclosure of the Invention

In one aspect, the invention is directed to a composition which comprises an apyrase and an annexin that binds phosphatidyl serine(PS) on the surface of activated platelets; said apyrase and annexin are administered in amounts that are together effective to inhibit thrombosis without increasing bleeding.

The combination of annexin and an apyrase may also be administered in the form of a single molecule wherein the apyrase and annexin are covalently coupled.

With respect to ease of production of such a covalently coupled combination, it is advantageous that the apyrase and annexin are covalently coupled through a linker peptide to form a fusion protein, especially wherein the linker peptide is resistant to hydrolysis in plasma and/or in cell culture supernatant.

The invention is also directed to recombinant production of the covalently coupled form of apyrase and annexin including the materials associated with such recombinant production and to compositions for use in treating a subject experiencing or at risk for thrombosis. The invention is defined in the claims.

### Brief Description of the Drawings

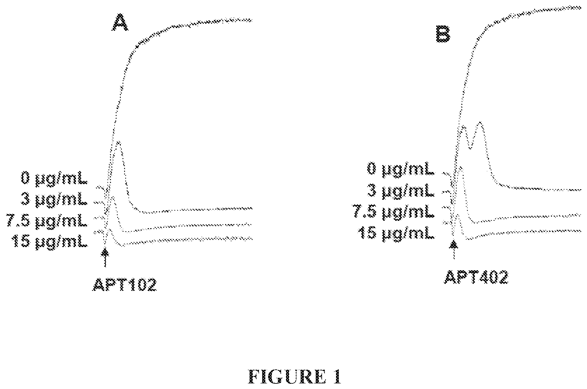

FIGS. 1A and 1B show the effect of apyrase APT102 used as such and of a fusion protein thereof, APT402, on platelet aggregation in an *ex vivo* reaction mixture comprising human platelet-rich plasma (PRP) activated with 20 µM ADP. Comparable inhibitory potency of ADP-induced human platelet aggregation by APT102 vs APT402 is shown. X axis is the assay time (6 min duration), Y axis is the light transmission. (A) APT102 dose-dependently inhibits platelet aggregation induced by 5 mM ADP. (B) APT402 dose-dependently inhibits platelet aggregation induced by 5 mM ADP. Arrows indicate addition of ADP and apyrases.
FIG. 2 shows the inhibitory activity on Factor X activation of APT402 vs annexin in PBMC. Comparable inhibitory potency of LPS-induced procoagulation activity by APT402 vs annexin V is shown. Peripheral blood mononuclear cells were incubated with LPS (1 mg/ml) to induce tissue factor expression and FX activation measured in the presence of FVII, FX and chromogenic substrate S2765.
FIG. 3 shows the synergistic effects of annexin V and APT102 on attenuation of thrombin generation. Shown are thrombograms of nanomolar thrombin that were generated in human platelet rich plasma activated with 20mM ADP. Treatment with APT402 or with APT102 plus annexin V shows comparable inhibitory effects. These results are superior to those obtained with APT102 or annexin V alone.
FIG. 4 shows PK analysis of APT402 in rabbits (n=2) for i.v. bolus injection at 0.4mg/kg.
FIG. 5 shows PK Analysis (ELISA) of APT402 with bolus injection at 0.2mg/kg followed by continuous IV infusion for 120 min in rabbits.
FIG. 6 shows inhibition of ADP-induced (50uM) platelet aggregation in rabbit platelet rich plasma with APT402 at 0.2mg/kg bolus followed by 24µg/kg/min for 120 min. The aggregation was effectively inhibited at 60 and 110 min and returned to the baseline level at 180 min.
FIG. 7 shows attenuation of thrombin generation with APT402 at 0.2mg/kg bolus followed by 24µg/kg/min infusion for 120 min in rabbits. Thrombin generation was effectively attenuated at 60 and 110 min but returned to the baseline level at 180 min.
FIG. 8A-C show APT402 is preferably localized at the thrombotic sites of injured arteries. A. APT402 was conjugated to the fluorescent dye (LS288); B. Fluorescent intensity in the injured artery of the rabbit treated with APT402 at 0.2mg/kg bolus followed by 12mg/kg/min for 120 min. Each bar represents the mean of 3 readings. C. APT402 preferably binds to thrombi.
FIG. 9 shows occlusion rate of control and various treatment groups in the electrical injury model (EIM) in rabbits. Treatment with APT402 at 12 or 24ug/kg/min completely prevented occlusion.
FIG. 10 shows arterial Thrombus Weight (mg) of the control and various treatment groups in the EIM in rabbits. Treatment with APT402 at 24ug/kg/min was more effective than any other treatment, alone or in combination.
FIG. 11 shows APT402 does not prolong bleeding time (BT) at doses that completely prevent occlusion (BT measurement ceiling: 5 min), while clopidogrel, LMWH, bivalirudin, and ticagrelor, alone or in combination with bivalirudin prolonged bleeding time.
FIG. 12 shows APT402 at effective doses does not affect prothrombin time in rabbits while bivalirudin, alone or in combination with ticagrelor increased prothrombin time.
FIG. 13 shows APT402 at effective doses does not affect partial thromboplastin time in rabbits while LMWH, bivalirudin, ticagrelor, alone or in combination with bivalirudin increased partial thromboplastin time.
FIG. 14 shows APT402 does not affect mean arterial pressure (millimeter of mercury; mmHg).
FIG. 15 shows APT402 does not affect heart rate (beats per minute; BPM).
FIG. 16A-D show effects of APT402 on sequelae of EIM on deep vein thrombosis (DVT).
FIG. 17 shows effect of APT402 on collagen deposition in EIM reduced DVT.

### Modes of Carrying out the Invention

The invention takes advantage of the unexpectedly synergistic combined action of antiplatelet and anticoagulant proteins, an apyrase and an annexin to prevent or treat both arterial and venous thrombosis. The combination solves problems associated with commercially available antithrombotics, and in particular has a more favorable profile with respect to avoiding enhanced bleeding as compared to these commercial antithrombotics. In addition, the invention includes unique fusion proteins wherein the apyrase and annexin are linked by additional amino acid sequence. When produced as a fusion protein, the invention includes advantageous forms thereof in terms of their expression levels when produced recombinantly and in terms of their resistance to proteolysis both in recombinant culture and in plasma. These features appear dependent on the linker provided between the annexin component and the apyrase component.

### The Components

In general, any apyrase that inhibits platelet activation and aggregation can be employed. A discussion of such apyrases can be found in US 7,390,485. Certain mutants of soluble forms of CD39L3 are particularly useful.

A particularly useful form of apyrase is the soluble form of CD39L3 the sequence of which is disclosed in US 7,390,485. Full length CD39L3 and its deduced amino acid sequence are SEQ ID NO: 55 and SEQ ID NO: 56, respectively therein. The soluble form is also disclosed in this patent and the sequence of the encoding nucleotides as SEQ ID NO: 59 and of the deduced amino acid sequence as SEQ ID NO: 60 therein.

The '485 patent also describes site directed mutagenesis of both soluble CD39 and soluble CD39L3. Particularly useful in the present invention are mutated forms of soluble CD39L3 wherein the amino acids at positions 67 and 69 are mutated. Two of these mutants of soluble CD39L3 are R67A T69R and R67G T69R. Soluble CD39L3 R67G T69R is a particularly preferred double mutant. An "enhanced" form of this sequence is disclosed in US 8,771,683 and the relevant nucleotide and amino acid sequences are reproduced herein as SEQ ID NOs:1and 2 respectively. This embodiment is designated APT102.

The annexin component useful in the invention is annexin V which binds phosphatidyl serine on activated platelets. The annexins are members of a large family of proteins with similar structures, but with various physiological activities. Any annexin that exhibits the above-mentioned property of annexin V may be used in the invention. The structures of these annexins including the native or optimized nucleotides sequences encoding them are known in the art. In some embodiments the annexin chosen will be of the same species as the subject of treatment.

### Production of the Invention Components

In general, it is convenient to produce the components useful in the invention using recombinant techniques. This is particularly the case for embodiments wherein the apyrase and annexin are linked covalently. Recombinant techniques for such proteins are well known in the art at this time and the recombinant production may be conducted in variety of cells and settings, including animal, plant and microbial cells in culture and as an *in situ* location in multicellular organisms. The invention therefore includes recombinant materials for production of these components, such as expression systems with suitable control sequences, vectors, host cells harboring these and the like.

### Administering Compositions of the Invention In Vivo

The compositions of the present invention may be administered to warm-blooded animals, including humans and other mammals as well as to domestic avian species. These may include companion or farm animals, for example. For treatment of human ailments, a qualified physician will determine how the compositions of the present invention should be utilized with respect to dose, schedule and route of administration using established protocols. A similar role is assumed by a veterinarian in other species. Such applications may also utilize dose escalation.

Preferably, the pharmaceutical compositions of the present invention are administered parenterally, *i.e.,* intraarterially, intravenously, intraperitoneally, subcutaneously, or intramuscularly. More preferably, the pharmaceutical compositions are administered intravenously or intraperitoneally by a bolus or infusional injection.

Pharmaceutical compositions of the invention are prepared according to standard techniques and may comprise water, buffered water, saline, glycine, dextrose, iso-osmotic sucrose solutions and the like, including glycoproteins for enhanced stability, such as albumin, lipoprotein, globulin, and the like. These compositions may be sterilized by conventional, well-known sterilization techniques. The resulting aqueous solutions may be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, and the like.

The concentration of the invention components in the pharmaceutical formulations can vary widely, such as from less than about 0.05%, usually at or at least about 2-5% to as much as 10 to 30% by weight and will be selected primarily by fluid volumes, viscosities, and the like, in accordance with the particular mode of administration selected. For example, the concentration may be increased to lower the fluid load associated with treatment.

Preferably, the pharmaceutical compositions of the present invention are administered intravenously. Dosage for the delivery vehicle formulations will depend on the ratio of drug to lipid and the administrating physician's opinion based on age, weight, and condition of the patient. One preferred protocol comprises a bolus IV administration followed by prolonged IV infusion.

In addition to pharmaceutical compositions, suitable formulations for veterinary use may be prepared and administered in a manner suitable to the subject. Preferred veterinary subjects include mammalian species, for example, non-human primates, dogs, cats, cattle, horses, sheep, and domesticated fowl. Subjects may also include laboratory animals, for example, in particular, rats, rabbits, mice, and guinea pigs.

### Indications

The compositions of the invention are useful in subjects where thrombosis is to be treated or prevented without engendering excessive bleeding. These include, without limitation, surgery, e.g., thoracic surgery including CABG and major surgery with high thrombotic risk or surgery or medical investigations where anti-thrombotic treatment is administered as prophylaxis against clotting on indwelling catheters or placement of devices (e.g. electrophysiological investigations, pacemaker implantations, percutaneous heart-valve placement or repair). Subjects to be treated with the invention compositions also include subjects with myocardial infarction, including STEMI, undergoing revascularization/reperfusion treatment with PCI or thrombolysis and subjects with stable or unstable coronary artery disease undergoing coronary revascularization with PCI. Suitable subjects are those with peripheral vascular disease undergoing revascularization by peripheral vessel-grafting or intra-luminal balloon angioplasty w/wo stenting, and subjects on chronic anti-thrombotic therapy, including those with atrial fibrillation and under treatment/prophylaxis for venous thromboembolism (VTE), and therefore a high risk of thrombotic complications, as a bridging strategy/peri-operative management during fasting and surgical procedures when subjects cannot sustain oral anti-thrombotic treatment.

The compositions are also useful as a prophylaxis or treatment for VTE - including deep vein thrombosis and pulmonary embolism - including prophylactic treatment related to orthopedic surgery and treatment of fractures, and immobilized subjects, subjects with acute or chronic illness (including cancer) and high risk of thrombosis. In addition the invention compositions are used to treat subjects with heparin induced thrombocytopenia (HIT) and subjects with acute ischemic stroke requiring additional anti-thrombotic treatment added on to standard-of-care, including before, during or after potential reperfusion treatment (but also when reperfusion treatment has not been used) as well as treatment or during pulmonary angioplasty procedures in subjects with chronic thromboembolic pulmonary disease / hypertension (CTEPH).

Stroke is defined as loss of neurological function due to brain ischemia or intracranial hemorrhages, including but may not be limited to, for prevention of tissue destruction and improved neurological function and outcome in conjunction with acute ischemic stroke; large and/or small vessel occlusion ischemic stroke in conjunction with reperfusion by thrombolysis or thrombectomy; embolic stroke; and prevention of recurrent stroke in patients with acute cerebral transitory ischemic attacks with high risk of stroke.

### Kits

The apyrases and annexin used in the invention compositions may be formulated separately in individual compositions or in the same composition or are covalently bound. Separate compositions can be administered separately to subjects simultaneously or sequentially. Kits that include, in separate containers, a first composition in a first container comprising an apyrase and, in a second container, a second composition comprising a suitable annexin can then be packaged into the kit.

The kit will also include instructions as to the mode of administration of the compositions to a subject, at least including a description of the ratio of amounts of each composition to be administered. In one embodiment, equimolar amounts are administered. However, the molar ratio of apyrase to annexin will depend on the choices for these components and can vary from 10:1 to 1:10 for apyrase:annexin. Alternatively, or in addition, the kit is constructed so that the amounts of compositions in each container is pre-measured so that the contents of one container in combination with the contents of the other represent the correct ratio. Alternatively, or in addition, the containers may be marked with a measuring scale permitting dispensation of appropriate amounts according to the scales visible. The containers may themselves be useable in administration; for example, the kit might contain the appropriate amounts of each composition in separate syringes. Formulations which comprise the pre-formulated correct ratio of therapeutic agents may also be packaged in this way so that the formulation is administered directly from a syringe prepackaged in the kit.

Unless defined otherwise, all terms of art, notations and other scientific terms or terminology used herein have the same meaning as is commonly understood by one of ordinary skill in the art to which this invention belongs. In some cases, terms with commonly understood meanings are defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art. As appropriate, procedures involving the use of commercially available kits and reagents are generally carried out in accordance with manufacturer defined protocols and/or parameters unless otherwise noted..

As used herein, "a" or "an" means "at least one" or "one or more."

The following examples are to illustrate, not limit the invention. Moreover, scientific discussions below of underlying mechanisms gleaned from the data are also not meant as limitations of the inventions described here.
Abbreviations: BT: bleeding time; aPTT: activated partial thromboplastin time; TCT: thrombin clotting time; ACT: activated clotting time; PT: prothrombin time; LMWH: Low molecular weight heparin; FX: Factor X; DVT: deep vein thrombosis; EIM: electrical injury model; IVC: inferior vena cava.

### EXAMPLE 1

### Apyrase annexin fusion construct and linker design

The apyrase agent used in these examples is a soluble apyrase agent made from a construct encoding soluble CD39L3 R67G T69R: with homogenized N-terminus as described in U.S. 8,771,683 designated APT102 (SEQ ID NO: 2) herein.

Nucleic acid-encoding annexin V was fused to the C-terminus of the apyrase agent with linkers of various lengths to optimize expression levels and prevent degradation and the various iterations were provided with a sequence encoding the mouse IgG kappa sequence as shown in SEQ ID NOs:3 and 4 herein and inserted into the expression plasmid pSecTag2c.

HEK 293 cells were stably transformed with the linearized expression plasmid that provides for the expression of the apyrase annexin V fusion proteins with variations in linker sequence.

Transformants were adapted to serum-free suspension culture and continually split to larger flasks. A typical suspension culture was inoculated at 0.5x10⁶ cells per mL and in 5 to 6 days HEK 293 cells grew typically over 3.5x10⁶ cells per mL. The cells were split every 3 - 4 days and fusion proteins in conditioned media were collected. The production of the apyrase annexin V fusion with 20 AA linker was scaled up to 3L spinner.

The proteins were purified to homogeneity using IMAC, Q and SP columns. The amino acid sequences of various linkers designed is shown below.
**>m0 (original design: cleaved by the protease)** 
GGCTCGACGAGCGGCGGGTCGACAAGTGGTGGATCTACTAGTGGCTCTGGATCCGGAATTGCT
G S T S G G S T S G G S T S G S G S G I A
cleavage |Anx V
**>m1 (cleaved by the protease)** 
GGCTCGACGAGCGGCGGGTCGACAAGTGGTGGATCTACTAGTGGCTCTGGATCCacAATTGCT
G S T S G G S T S G G S T S G S G S T I A
|Anx V
**>m2 (no cleavage was detected)** 
GGCTCGACGAGCGGCGGGTCGACAAGTGGTGGATCTACTAGTGGCTCTGGAgCCacAATTGCT
G S T S G G S T S G G S T S G S G A T I A
|Anx V
**>m3 (no cleavage was detected)**

GGCTCGACGAGCGGCGGGTCGACAAGTGGTGGATCTACTAGTGGCTCTGGAcagacAATTGCT
G S T S G G S T S G G S T S G S G Q T I A
|Anx V

For the originally designed linker, m0, approximately 50% of the purified protein was degraded in the linker region by the protease activity present in the culture medium. Further study shows that low pH (*e.g.* 5.0) accelerated the degradation compared to pH of 7.4. Protease resistant fusion protein was designed by introducing G to T substitution as shown in m1, which was still susceptible to the protease degradation when the pH of the clarified supernatant was reduced to 5 with sodium citrate. When this mutation was combined with the replacement of serine with alanine or glutamine as shown in m2 and m3, degradation was minimized.

It was also shown that a 20 amino acid linker was most advantageous based on comparison of expression levels.

The expression levels were compared with a flexible linkers of 5, 10, 15 and 20 amino acid residues in length or a rigid linker of 9 amino acid residues.
5AA. GSTSG
10AA. (GSTSG)x2
15AA. (GSTSG)x3
20AA. (GSTSG)x3 SGATI
9AA. PAPAPAPAP

The proteins from clarified supernatants of the conditioned media as described above were separated on SDS polyacrylamide gel electrophoresis. The analysis shows the fusion protein expression was positively correlated with the length of the linkers with 20AA resulting in the highest expression. The rigid linker construct also resulted in a lower expression than the variant with 20AA flexible linker. The amino acid and nucleotide sequences of this 20AA linker are shown in SEQ ID NO:3.

The final construct used in Examples 2-10 has the nucleotide sequence SEQ ID NO: 7 wherein the signal sequence encoded bovine alpha lactalbumin signal peptide. The encoded fusion of apyrase-linker-annexin V is designated APT402.

### EXAMPLE 2

### Expression and purification of APT402 from CHO cells

The apyrase agent in the construct for production of the fusion protein APT402 is APT102. The apyrase annexin V fusion with 20 AA linker described in Example 1 was produced in CHO cells with the signal sequence of the bovine α-lactalbumin signal peptide from a construct shown as SEQ ID NO: 7. As noted, the apyrase-linker-annexin protein was designated as APT402. The production cell lines were made by four rounds of transduction of the CHO parental cell line with retrovector made from the Catalent (Madison, USA) and expression retrovector plasmid. The pooled population of transduced cells was named CHO-S-APT402-R 4X pool. Samples of the pooled population cell lines were cryopreserved.

For 10L production, cell line CHO-S-APT402-R 4X pool was passaged every 3-4 days during the exponential growth phase for scale-up for the 10L Braun bioreactors in PFCHO LS (HyClone). Cells were inoculated at a cell density of approximately 300,000-400,000 cells/ml in PFCHO LS (HyClone) medium into two 10L bioreactors. Fed-batch supplements used for this study were HyClone PS307 (12% (w/v) solutions), AGT CD CHO 5X Feed Medium Complete (Invitrogen), 20% glucose solution, 200 mM L-glutamine, 50X solution of L-Asparagine (15 g/L)/L-Serine (10 g/L), 50X solution of L-Tyrosine (4 g/L)/L-Cystine (2 g/L).

ANX chromatography. The harvest of 10LBRX-3380-4Xpool-001 from 10L vessel occurred on day 12 of culture. The column was washed for equilibration with 10mM Tris-Cl, pH 7.4. The Triton-treated media was diluted with an equal volume (6.60L) of WFI to prepare the ANX load. The load (13.2L) was applied to the column. The column was then washed with 10 mM Tris, 100mM NaCl, pH 7.4 and 3L of this wash was collected. The APT402 fraction was eluted with 10 mM Tris, 270mM NaCl, pH 7.4 and collected (2.37L).

SP Sepharose Chromatography. The column was sanitized with 1M NaOH for over an hour, and then the column was washed with WFI. The column was equilibrated in 10mM Tris, 50mM NaCl, 20mM CaCl₂, pH 7.4. The load (ANX elute + 9.4L of 10mM Tris, pH 7.4 + 34.5g of CaCl₂ added and stirred 20minutes, 11.75L) was applied to the column and the flowthrough, plus wash, collected (~12L). The column was washed with 10mM Tris, 50mM NaCl, pH 7.4 and the baseline was re-established. The column was eluted with 10mM Tris, 300mM NaCl, pH 7.4 and the elution peak collected (1.0L) as a clear colorless solution.

Heparin Hyper D Chromatography. The column was washed with 10mM Tris, 1M NaCl, pH 7.4. The column was then equilibrated in 10mM Tris, pH 7.4. The load (Buffer Exchanged SP Pool, 0.88L) was applied to the column and the flowthrough, plus wash, collected (1.40L). The column was stripped of remaining protein with 10mM Tris, 1 M NaCl, pH 7.4 and this too, was collected (~100mL). The chromatogram is shown below. The Heparin flowthrough volume (1.40L) was buffer exchanged in discontinuous mode using a Masterflex pump and Pellicon Polysulfone 10K membrane into 10mM Tris, 150mM NaCl, pH 7.4. The SP pool was concentrated ~7x to 200mL, diluted ~10x with 10mM Tris, 150mM NaCl, pH 7.4 to 2.0L, concentrated again to 200mL and diluted again ~10x to 2.0L with 10mM Tris, 150mM NaCl, pH 7.4. Following a third concentration to ~100mL, the filter was flushed with 200mL of 10mM Tris, 150mM NaCl, pH 7.4 and this was added to the retentate. The final volume of the concentrate was 300mL and ~4.9L of permeate was collected (~24.5x the initial concentrate volume). The retentate solution was further concentrated using an Amicon stirred cell apparatus using a 10kD regenerated cellulose membrane.

The protein was purified to homogeneity. The final recovery yield was ~54% overall. Bioburden was 0 CFU and endotoxin in the formulated bulk was assayed at 1.0 < X < 2.0EU/mL or 0.6 < X < 1.2EU/mg.

### EXAMPLE 3

### APT402 exhibited comparable ex vivo anti-platelet activity to APT102 and comparable ex vivo Factor X activation inhibition to annexin V

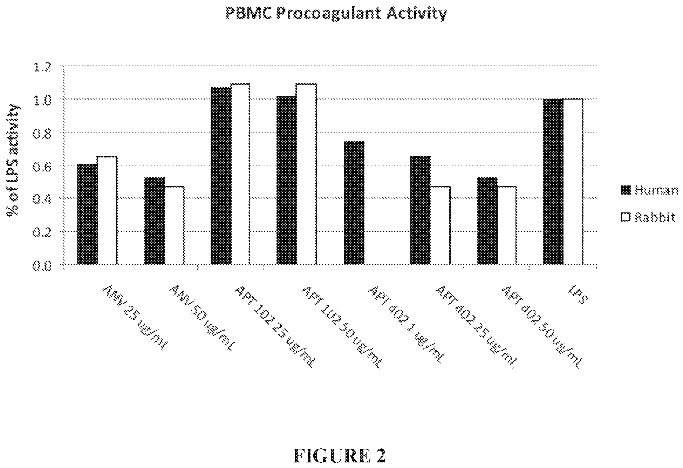

APT402 was designed to maintain the enzymatic and biological activity of both APT102 and annexin V. Using malachite green assay, we found the enzymatic kinetic parameters of APT402 for ATP and ADP were comparable to those of APT102. Similarly, APT402 inhibited ADP-induced human platelet aggregation with comparable potency to APT102 (Figure 1). The inhibitory activity on FX activation was assayed using peripheral blood mononuclear cells (PBMC) purified from normal human donors and anesthetized rabbits by standard methods. There was a similar dose-dependent reduction in LPS-induced FX activation for both APT402 and annexin V in rabbit and in human PBMCs (Figure 2). As expected, APT102 had no effect on PBMC pro-coagulant activity.

### EXAMPLE 4

### APT402 exhibited synergistic inhibition of thrombin generation from activated human plasma rich platelets compared to APT102 and annexin V alone

Thrombin generation in citrated human platelet rich plasma (PRP) was quantified by calibrated automated thrombography (CAT) using the Thrombinoscope system (Synapse), according to methods developed by Hemker et al. Aliquots of PRP were incubated with agents for 15 min at room temperature, and then for 5 min at 37°C in wells of 96-well plates. Thrombin generation was initiated by addition of 0.5 pM tissue factor and CaCl₂ to 16.7 mM and monitored in a microtiter plate fluorometer (Fluoroskan Ascent, Thermo Electron Corp., Vantaa, Finland). Thrombograms (nM thrombin generated vs. time) were generated using the Thrombinoscope software, as were key thrombin generation parameters: lag time to onset of thrombin generation (min), peak thrombin concentration (nM), time to peak thrombin (min), and endogenous thrombin potential (ETP; integrated area under the thrombogram curve).

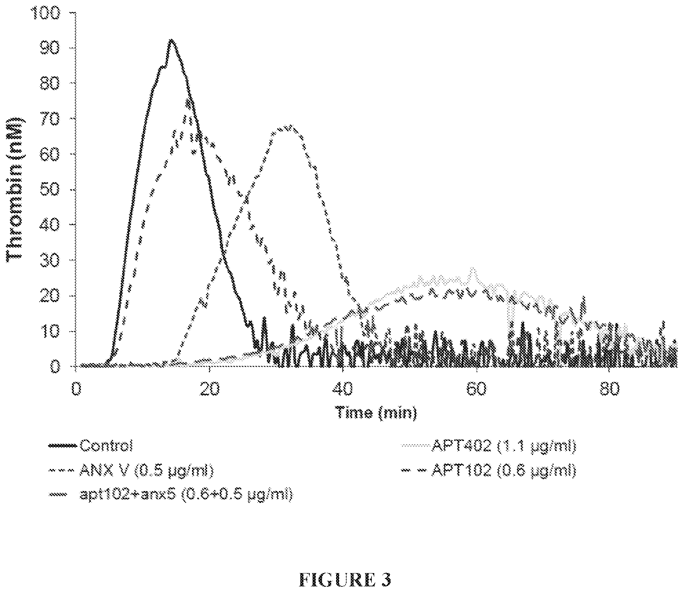

The effects of APT102, annexin V, APT102 + annexin V, and APT402 at equal molar concentrations on thrombin generation from 20uM ADP-activated human platelets were compared using CAT assays (Figure 3). Annexin V significantly increased time to peak thrombin while modestly decreasing peak thrombin concentration. APT102 had modest inhibitory effect on both parameters. However, APT102 plus annexin V synergistically inhibited thrombin generation which significantly increased time to peak thrombin by 4-fold while significantly decreasing peak thrombin by 3-fold. These features were mimicked by APT402.

### EXAMPLE 5

### APT402 displayed a fast onset of action after single bolus dosing in healthy rabbits

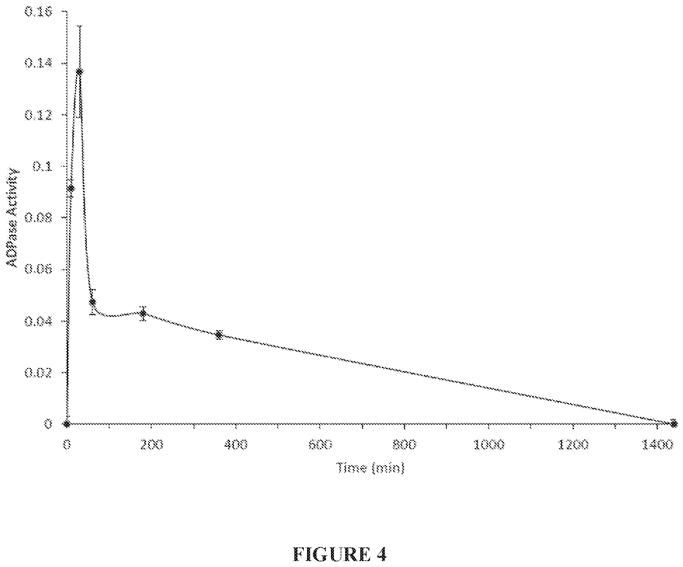

APT402 was injected IV as a single bolus (0.40 mg/kg) in two rabbits. Pharmacokinetic modeling showed best fits to biphasic exponential curves for ADPase activity (Figure 4). The maximal activity was detected in the plasma 30 min after administration. The distribution phase and elimination half-life (t_{½}) of APT402 were 30 min and 6 h, respectively, which are significantly extended compared to the distribution half-life of 5 min and elimination half-life of 20 min (18-fold) for annexin V. Administration of APT402 displayed a fast onset of action inhibiting 95% of 20µM ADP-induced *ex vivo* platelet aggregation by 10 min after IV dosing and 90% and 80% inhibition at 1 and 6 h after dosing, respectively. These data suggest that with relative short half life, APT402 should be administered as a bolus followed by IV infusion to ensure consistent attenuation of thrombosis.

### EXAMPLE 6

### Continuous IV infusion of APT402 provided rapid onset and off-set of action in healthy rabbits

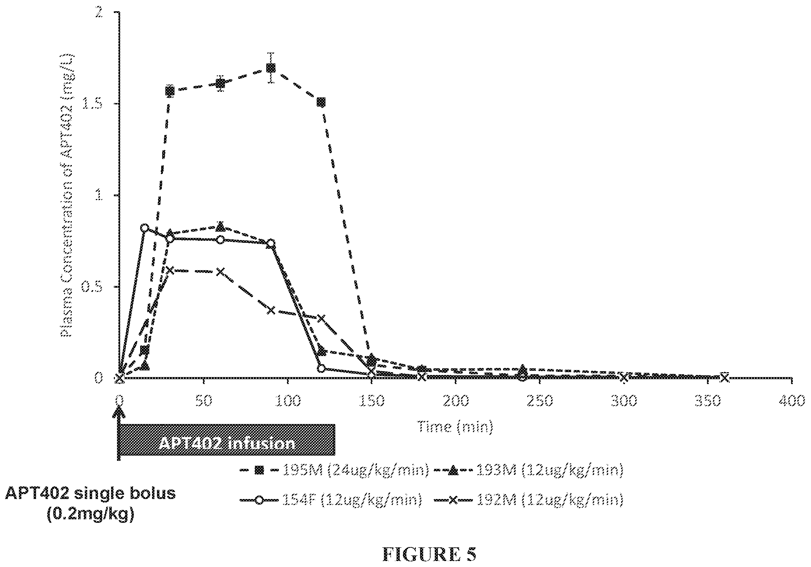

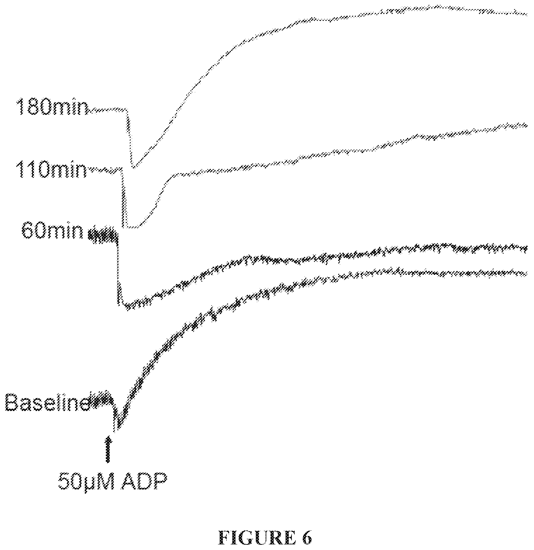

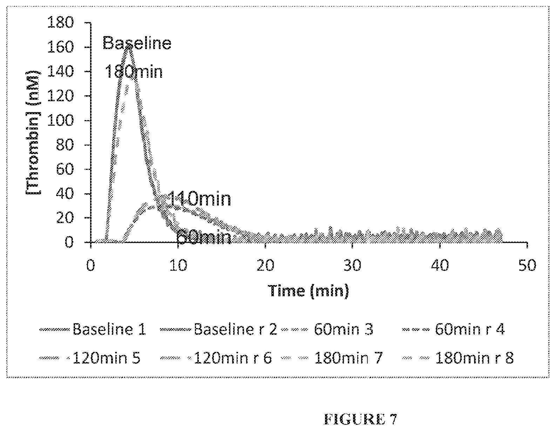

The preliminary data indicate APT402 had a short distribution half life. APT402 was then injected IV as a single bolus (0.2 mg/kg) followed by IV infusion at 12 and 24 µg/kg/min for 120 min in rabbits. ELISA data show that APT402 was detectable in the plasma by 30 min after administration. The APT402 concentration, inhibition of ADP-induced platelet aggregation and thrombin generation was maintained up to 120 min during infusion, then significantly decreased 60 min after discontinuation of APT402 infusion (Figure 5, 6, 7). There were no differences between APT402-treated and control animals regarding bleeding time, ACT, PT, aPTT, or platelet counts. The data suggest that optimal treatment with APT402 will be achieved with 30 min pretreatment to ensure attenuation of both local and systemic thrombosis.

### EXAMPLE 7

### APT402 is preferentially localized at the thrombotic sites of injured arteries in rabbits

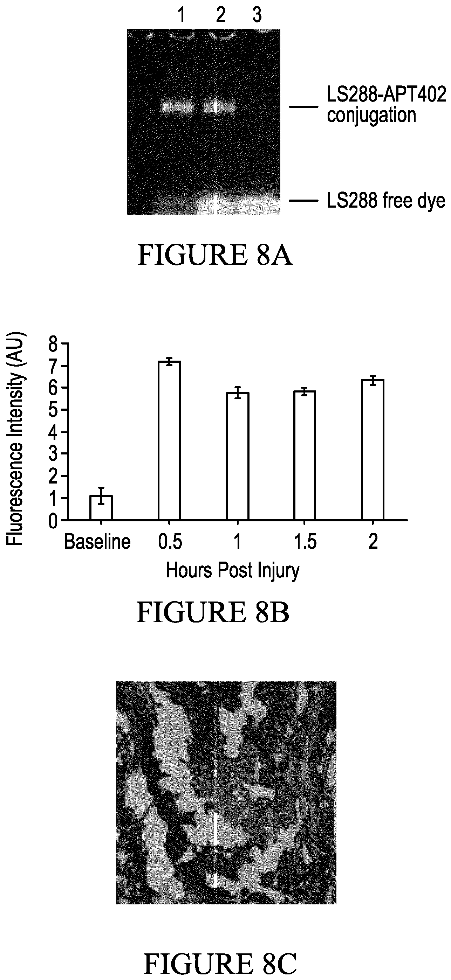

A near infra-red (NIR) fluorescent dye (LS288, Ex/Em 773/793) in methanol was conjugated to the functional amines of APT402 (~1:1). The purified bioconjugate showed one fluorescent band (Figure 8A). For *in vivo* imaging of the thrombus by fluorescent molecular tomography (FMT), a custom built, fiber-based, portable, video-rate system was used as described (CITE). Anodal current was applied to the carotid needle electrode to initiate thrombosis 30 min after the bolus of APT402 (NIR-labeled and non-labelled APT402 followed by IV infusion at 12 µg/kg/min that maintained patency of the injured carotid for 2 h. The average fluorescence values and confidence intervals for three rabbits are shown in Fig. 8B. The injury site was associated with approximately 6-7-fold increase in fluorescence after baseline due to thrombus forming around the transluminal needle electrode. Importantly, APT402 was found to be bound to thrombus (Figure 8C). In contrast, there was no increased signal in the non-injured control carotid (data not shown). Thus, APT402 is targeted specifically to the site of arterial injury and thrombosis. The tissue distribution of the NIR-labeled APT402 indicates that majority of the label was taken up by the liver and spleen.

### EXAMPLE 8

### APT402 was more effective in attenuating arterial thrombosis in rabbits than other antiplatelet or anticoagulant agents, alone or in combination, without increasing bleeding risk

Rabbits were randomized into the following eleven groups with the treatments initiated 30 min before electrical injury.

| | |
|---|---|
| Group 1(n=10): | Control saline IV infusion for 2h; |
| Group 2 (n=6): | Clopidogrel at 4 mg/kg oral and IV saline infusion; |
| Group 3 (n=6): | low molecular weight heparin (LMWH) at 0.7 mg/kg IV bolus followed by 1 mg/kg/h IV infusion; |
| Group 4 (n=7): | Bivalirudin at 5 mg/kg IV bolus followed by 5 mg/kg/h IV infusion; |
| Group 5: (n=6): | APT102 at 0.3 mg/kg IV bolus (n=2) or 1.0 mg/kg IV bolus (n=4); |
| Group 6 (n=6): | APT402 at 0.2 mg/kg IV bolus followed by 4µg/kg/min IV infusion for 2h initiated 30 min before electrical injury; |
| Group 7 (n=6): | APT402 at 0.2 mg/kg IV bolus followed by 12 µg/kg/min iv infusion for 2h started at the time of injury. |
| Group 8 (n=6): | APT402 at 0.2 mg/kg IV bolus followed by 24 µg/kg/min iv infusion for 2h started at the time of injury. |
| Group 9 (n=6): | Ticagrelor at 10 mg/kg in hydroxypropyl methylcellulose (HPMC) oral and IV saline infusion. |
| Group 10 (n=6): | Ticagrelor at 10 mg/kg in HPMC oral and Bivalirudin at 5 mg/kg IV bolus followed by 5 mg/kg/h IV infusion. |
| Group 11 (n=6): | HPMC oral and IV saline infusion. |

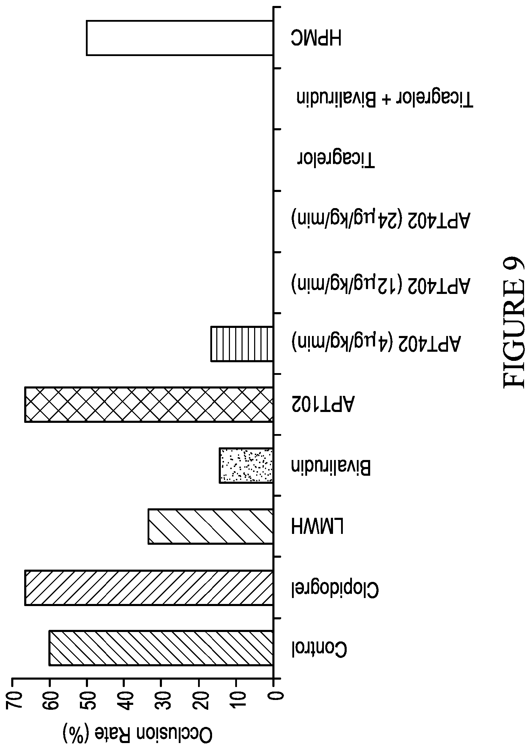

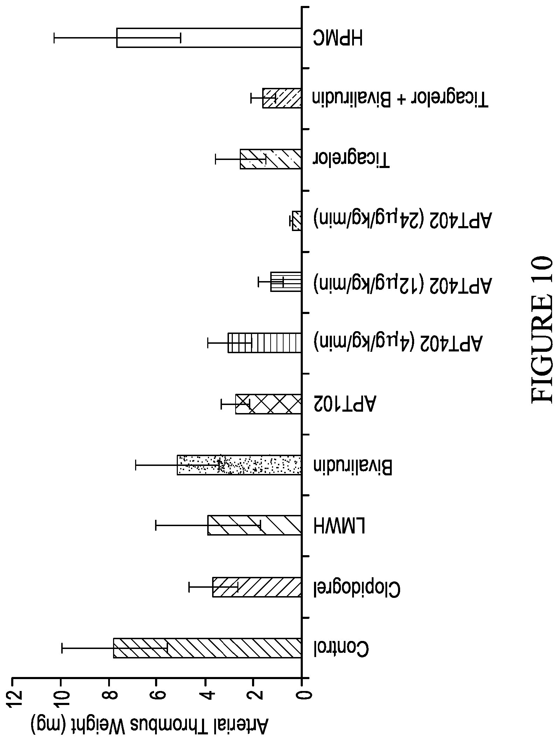

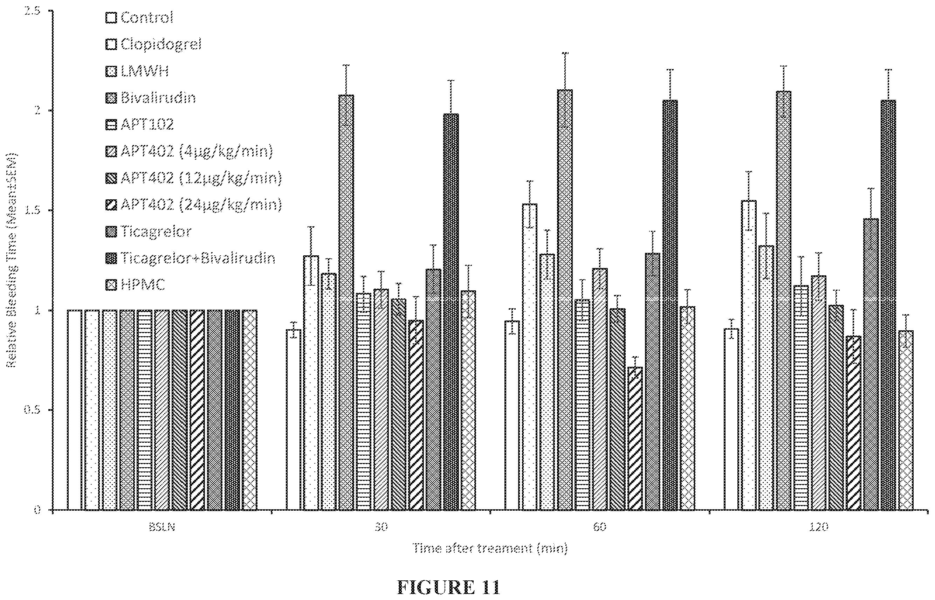

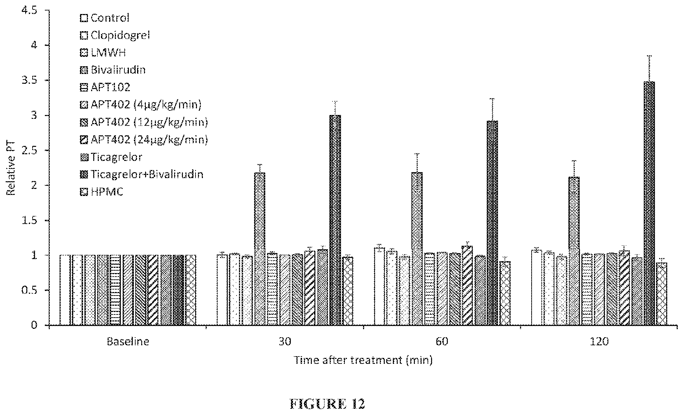

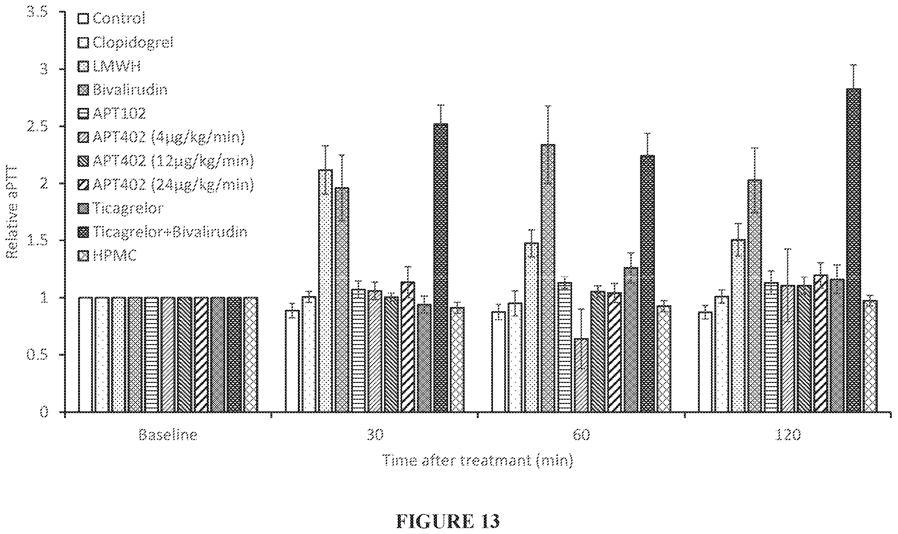

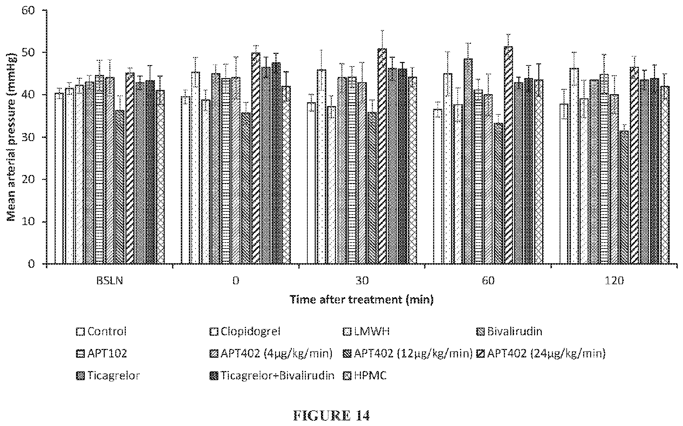

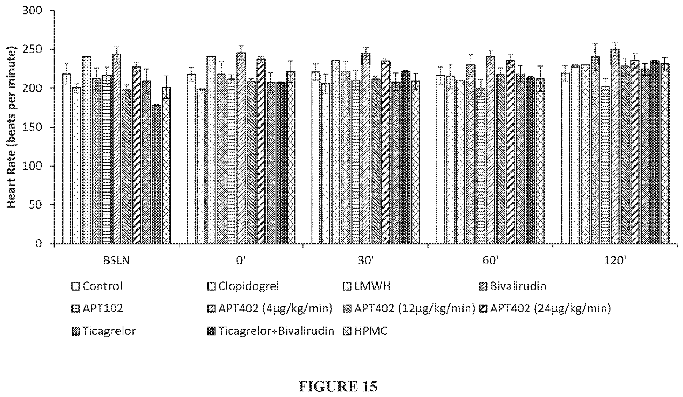

Electrical injury generated occlusion in 60% of the rabbits treated with saline within 2 h. Average thrombosis weight was 7.8 mg. Treatment with APT402 at 12 or 24 ug/kg/min, ticagrelor, alone or in combination with angiomax completely prevented occlusion (Figure 9). The thrombosis weight was dose-dependently reduced by APT402 (Figure 10). Strikingly, treatment with APT402 at 24ug/kg/min resulted in the lowest thrombus weight than any other treatments, including the combination of ticagrelor plus bivalirudin. Meanwhile, APT402 did not affect bleeding time (Figure 11), prothrombin time (Figure 12), partial thromboplastin time (Figure 13), blood pressure (Figure 14) or heart rate (Figure 15).

### EXAMPLE 9

### APT402 attenuated venous thrombosis without increasing bleeding or inducing hypocoagulability in an acute murine electrical injury model (EIM) of venous thrombosis, i.e., deep vein thrombosis (DVT)

An electrical injury model of venous thrombosis was used for acute study (Diaz JA et al. Thromb Haemost. 104: 366-375, 2010.

Mice were randomized into the following four groups (n = 10/group).

| |
|---|
| 1) Control: Saline ip on days 1 and 2; |
| 2) LMWH: 6 mg/kg/day sc on days 1 and 2; |
| 3) APT402 low dose (LD): 0.2 mg/kg ip bolus followed by 0.03 mg/kg/h sc infusion for 48h; and |
| 4) APT402 high dose (HD): 0.2 mg/kg ip bolus followed by 0.09 mg/kg/h sc infusion for 48h. |

The EIM consistently generated IVC thrombosis in all mice with the mean thrombus weight of 22.5 mg. Thrombus weight, BT, APTT and TCT were measured 48h post DVT induction.

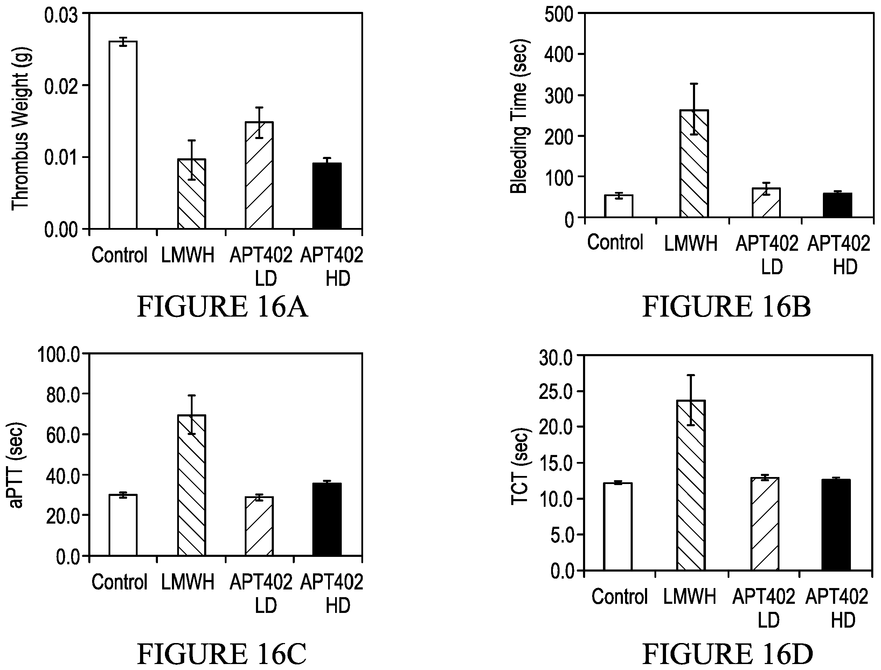

Treatment with LMWH reduced thrombus weight by 57% compared to controls and was associated with a significant prolongation of bleeding time by 3-fold, aPTT by 2.5-fold, and TCT by 2-fold (Figure 16).

APT402 dose-dependently reduced thrombus weight by 44% and 65% at the low and high doses, respectively. Importantly, APT402 did not cause detectable prolongation of BT, aPTT or TCT (Figure 16). Similarly, APT402 at 1.0 mg/kg ip bolus daily for 2 days also reduced thrombus weight by 53% without increasing bleeding time.

These data suggest that APT402 is safe and will be more effective for DVT treatment than enoxaparin due to lack of dose-limiting bleeding.

### EXAMPLE 10

### APT402 decreased fibrosis in a chronic murine electrolytic injury model (EIM) of DVT without increasing bleeding

In a chronic fibrotic study, mice were randomized and blinded into the following groups (n = 5-12/group).

| Control: | |
|---|---|
| | Healthy (n=5); |
| | placebo (n=12): Saline ip daily for 14 days; |
| | APT402: 1.0 mg/kg ip bolus daily for 14 days; and |
| | APT102: 1.0 mg/kg ip bolus daily for 14 days. |

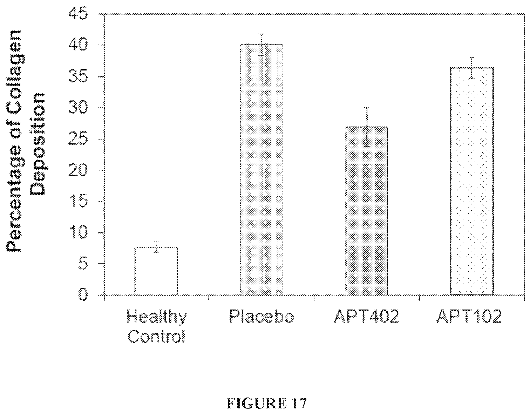

Collagen deposition was stained with Masson's trichrome 14 days post DVT induction. The EIM consistently induced IVC fibrosis in all mice and increased mean collagen deposition by approximately 6-fold compared to the healthy mice (Figure 17).

Treatment with APT402 and APT102 significantly reduced collagen deposition by 33% and 11% respectively. There was one death in the placebo group, while no deaths or increased bleeding or gross side effects were observed in the APT402 and APT102 groups.

These data suggest that APT402 is safe and may be an effective treatment for post-thrombotic syndrome.

### SEQUENCE LISTING

Soluble CD39L3 R67G T69R (APT102)
SEQ ID NO:1 - nucleotide
SEQ ID NO:2 - amino acid 
gaggtcctccctccaggactgaagtatggtattgtgctggatgccgggtcttcagggacc
 E  V  L  P  P  G  L  K  Y  G  I  V  L  D  A  G  S  S  G  T
cgcgtctacgtgtatcaatggccagcagaaaaagagaataataccggagtggtcagtcaa
 R  V  Y  V  Y  Q  W  P  A  E  K  E  N  N  T  G  V  V  S  Q
accttcaaatgtagtgtgaaaggctctggaatctccagctatggaaataacccccaagat
 T  F  K  C  S  V  K  G  S  G  I  S  S  Y  G  N  N  P  Q  D
gtccccagagcctttgaggagtgtatgcaaaaagtcaaggggcaggttccatcccacctc
 V  P  R  A  F  E  E  C  M  Q  K  V  K  G  Q  V  P  S  H  L
cacggatccacccccattcacctgggagccacggctgggatgcgcttgctgaggttgcaa
 H  G  S  T  P  I  H  L  G  A  T  A  G  M  R  L  L  R  L  Q
aatgaaacagcagctaatgaagtccttgaaagcatccaaagctacttcaagtcccagccc
 N  E  T  A  A  N  E  V  L  E  S  I  Q  S  Y  F  K  S  Q  P
tttgactttaggggtgctcaaatcatttctgggcaagaagaaggggtatatggatggatt
 F  D  F  R  G  A  Q  I  I  S  G  Q  E  E  G  V  Y  G  W  I
acagccaactatttaatgggaaatttcctggagaagaacctgtggcacatgtgggtgcac
 T  A  N  Y  L  M  G  N  F  L  E  K  N  L  W  H  M  W  V  H
ccgcatggagtggaaaccacgggtgccctggacttaggtggtgcctccacccaaatatcc
 P  H  G  V  E  T  T  G  A  L  D  L  G  G  A  S  T  Q  I  S
ttcgtggcaggagagaagatggatctgaacaccagcgacatcatgcaggtgtccctgtat
 F  V  A  G  E  K  M  D  L  N  T  S  D  I  M  Q  V  S  L  Y
ggctacgtatacacgctctacacacacagcttccagtgctatggccggaatgaggctgag
 G  Y  V  Y  T  L  Y  T  H  S  F  Q  C  Y  G  R  N  E  A  E
aagaagtttctggcaatgctcctgcagaattctcctaccaaaaaccatctcaccaatccc
 K  K  F  L  A  M  L  L  Q  N  S  P  T  K  N  H  L  T  N  P
tgttaccctcgggattatagcatcagcttcaccatgggccatgtatttgatagcctgtgc
 C  Y  P  R  D  Y  S  I  S  F  T  M  G  H  V  F  D  S  L  C
actgtggaccagaggccagaaagttataaccccaatgatgtcatcacttttgaaggaact
 T  V  D  Q  R  P  E  S  Y  N  P  N  D  V  I  T  F  E  G  T
ggggacccatctctgtgtaaggagaaggtggcttccatatttgacttcaaagcttgccat
 G  D  P  S  L  C  K  E  K  V  A  S  I  F  D  F  K  A  C  H
gatcaagaaacctgttcttttgatggggtttatcagccaaagattaaagggccatttgtg
 D  Q  E  T  C  S  F  D  G  V  Y  Q  P  K  I  K  G  P  F  V
gcttttgcaggattctactacacagccagtgctttaaatctttcaggtagcttttccctg
 A  F  A  G  F  Y  Y  T  A  S  A  L  N  L  S  G  S  F  S  L
gacaccttcaactccagcacctggaatttctgctcacagaattggagtcagctcccactg
 D  T  F  N  S  S  T  W  N  F  C  S  Q  N  W  S  Q  L  P  L
ctgctccccaaatttgatgaggtatatgcccgctcttactgcttctcagccaactacatc
 L  L  P  K  F  D  E  V  Y  A  R  S  Y  C  F  S  A  N  Y  I
taccacttgtttgtgaacggttacaaattcacagaggagacttggccccaaatacacttt
 Y  H  L  F  V  N  G  Y  K  F  T  E  E  T  W  P  Q  I  H  F
gaaaaagaagtggggaatagcagcatagcctggtctcttggctacatgctcagcctgacc
 E  K  E  V  G  N  S  S  I  A  W  S  L  G  Y  M  L  S  L  T
aaccagatcccagctgaaagccctctgatccgtctgcccatagaaccacctgtctga
 N  Q  I  P  A  E  S  P  L  I  R  L  P  I  E  P  P  V  -
APT402 with optimized codons and mouse Ig kappa signal peptide sequence SEQ ID NO:3 - nucleotide
SEQ ID NO:4 - amino acid 
atggagacagacacactcctgctatgggtactgctgctctgggttccaggttccactggt
 M  E  T  D  T  L  L  L  W  V  L  L  L  W  V  P  G  S  T  G
|mouse Ig kappa leader sequence ->

gacgcgcccgaggtgctgcccccaggcctgaagtacgggatcgtcctggacgccggaagc
 D  A  P  E  V  L  P  P  G  L  K  Y  G  I  V  L  D  A  G  S
         |soluble CD39L3 R69G T69R ->

tccggtacccgggtctatgtgtaccagtggcccgccgaaaaggagaacaatacaggcgtg
 S  G  T  R  V  Y  V  Y  Q  W  P  A  E  K  E  N  N  T  G  V

gtgtcacaaaccttcaagtgttcagtcaaaggtagtggaatttcttcatatgggaacaat
 V  S  Q  T  F  K  C  S  V  K  G  S  G  I  S  S  Y  G  N  N

ccccaggatgtgcctagggcatttgaggaatgtatgcagaaagtcaagggccaggtccca
 P  Q  D  V  P  R  A  F  E  E  C  M  Q  K  V  K  G  Q  V  P

tctcatctgcatggttcaactcccatccatctgggtgccactgcaggaatgaggctgctg
 S  H  L  H  G  S  T  P  I  H  L  G  A  T  A  G  M  R  L  L

cggctgcagaatgaaaccgcagccaacgaggtcctggaatccattcaaagttacttcaag
 R  L  Q  N  E  T  A  A  N  E  V  L  E  S  I  Q  S  Y  F  K

tcccaaccttttgacttccgaggcgctcaaattatcagtggtcaggaagagggagtctat
 S  Q  P  F  D  F  R  G  A  Q  I  I  S  G  Q  E  E  G  V  Y

ggctggatcaccgcaaactacctgatgggtaattttctggagaagaacctgtggcatatg
 G  W  I  T  A  N  Y  L  M  G  N  F  L  E  K  N  L  W  H  M

tgggtccatccccacggtgtcgaaacaactggagccctggatctgggcggtgccagtaca
 W  V  H  P  H  G  V  E  T  T  G  A  L  D  L  G  G  A  S  T

cagatctcatttgtcgctggtgagaaaatggacctgaacaccagcgatatcatgcaagtc
 Q  I  S  F  V  A  G  E  K  M  D  L  N  T  S  D  I  M  Q  V

tctctgtatggatacgtctatactctgtacacccactctttccaatgttacggtcgcaac
 S  L  Y  G  Y  V  Y  T  L  Y  T  H  S  F  Q  C  Y  G  R  N

gaagccgagaagaaattcctggctatgctgctgcaaaattctcctaccaagaatcatctg
 E  A  E  K  K  F  L  A  M  L  L  Q  N  S  P  T  K  N  H  L

actaatccttgctatcccagggactacagtattagctttactatgggccacgtgttcgat
 T  N  P  C  Y  P  R  D  Y  S  I  S  F  T  M  G  H  V  F  D

tccctgtgcacagtcgaccagcggcccgagtcttacaatcccaacgatgtgatcaccttc
 S  L  C  T  V  D  Q  R  P  E  S  Y  N  P  N  D  V  I  T  F

gagggcactggtgacccctcactgtgcaaggagaaagtcgcaagtatcttcgactttaag
 
E  G  T  G  D  P  S  L  C  K  E  K  V  A  S  I  F  D  F  K

gcttgccacgatcaagaaacttgctcctttgatggcgtctatcaaccaaaaatcaagggc
 A  C  H  D  Q  E  T  C  S  F  D  G  V  Y  Q  P  K  I  K  G

ccatttgtcgccttcgccgggttctattataccgcatccgccctgaacctgtctggtagc
 P  F  V  A  F  A  G  F  Y  Y  T  A  S  A  L  N  L  S  G  S

ttcagtctggacacttttaattcaagtacatggaacttctgctctcagaattggagtcaa
 F  S  L  D  T  F  N  S  S  T  W  N  F  C  S  Q  N  W  S  Q

ctgcccctgctgctgcccaaattcgatgaggtgtacgctaggagttattgtttctcagca
 L  P  L  L  L  P  K  F  D  E  V  Y  A  R  S  Y  C  F  S  A

aactatatctaccatctgtttgtgaatggctacaaattcacagaggagacatggccacaa
 N  Y  I  Y  H  L  F  V  N  G  Y  K  F  T  E  E  T  W  P  Q

attcactttgagaaagaggtcggaaattcatctattgcatggagcctggggtacatgctg
 I  H  F  E  K  E  V  G  N  S  S  I  A  W  S  L  G  Y  M  L

agcctgactaatcagattcccgctgaatcacccctgatccgactgcctattgaaccaccc
 S  L  T  N  Q  I  P  A  E  S  P  L  I  R  L  P  I  E  P  P

gtcggctcgacgagcggcgggtcgacaagtggtggatctactagtggctctggatccgga
 V  G  S  T  S  G  G  S  T  S  G  G  S  T  S  G  S  G  S  G
   |20 amino acid linker ->

attgctcaagtgctgaggggaactgtgactgacttcccaggcttcgatgaaagggcagac
 I  A  Q  V  L  R  G  T  V  T  D  F  P  G  F  D  E  R  A  D
   |Annexin V ->

gctgagactctgcgcaaagcaatgaagggtctgggtacagatgaagagagtattctgaca
 A  E  T  L  R  K  A  M  K  G  L  G  T  D  E  E  S  I  L  T

ctgctgactagtcgctccaacgcccaaagacaggagattagcgcagcattcaaaactctg
 L  L  T  S  R  S  N  A  Q  R  Q  E  I  S  A  A  F  K  T  L

ttcggtagggatctgctggacgatctgaaatctgaactgaccgggaaattcgagaaactg
 F  G  R  D  L  L  D  D  L  K  S  E  L  T  G  K  F  E  K  L

atcgtggccctgatgaaaccaagtcggctgtatgatgcatacgaactgaagcacgctctg
 I  V  A  L  M  K  P  S  R  L  Y  D  A  Y  E  L  K  H  A  L

aagggagccggcactaatgagaaggtgctgacagaaattatcgcatctcgaacccccgag
 K  G  A  G  T  N  E  K  V  L  T  E  I  I  A  S  R  T  P  E

gaactgcgcgctatcaagcaagtgtatgaagaggaatatggttccagcctggaggacgat
 E  L  R  A  I  K  Q  V  Y  E  E  E  Y  G  S  S  L  E  D  D

gtcgtcggggatacctcaggttattaccaacgcatgctggtggtgctgctgcaggccaac
 V  V  G  D  T  S  G  Y  Y  Q  R  M  L  V  V  L  L  Q  A  N

agggaccctgacgctggcatcgatgaagcccaggtggaacaagacgcccaggcactgttc
 
R D P D A G I D E A Q V E Q D A Q A L F

caagcaggggagctgaagtgggggaccgacgaggaaaagtttatcaccatctttggaact
 Q A G E L K W G T D E E K F I T I F G T

cggagtgtgagccatctgcgcaaagtctttgacaagtatatgacaatttccggcttccaa
 R S V S H L R K V F D K Y M T I S G F Q

atcgaggaaaccattgatcgagagactagcggcaatctggaacagctgctgctggctgtg
 I E E T I D R E T S G N L E Q L L L A V

gtgaaatcaatcagatcaattcctgcatatctggcagagacactgtattacgcaatgaaa
 V K S I R S I P A Y L A E T L Y Y A M K

ggagccggaaccgacgatcatactctgattagagtcatggtcagtcggtccgagatcgac
 G A G T D D H T L I R V M V S R S E I D

ctgtttaacattagaaaggagttccgcaagaattttgcaacttccctgtactcaatgatc
 L F N I R K E F R K N F A T S L Y S M I

aaaggcgatacctctggtgactacaagaaagctctgctgctgctgtgcggcgaggatgac
 K G D T S G D Y K K A L L L L C G E D D

taa
 -
20AA linker
SEQ ID NO:5 - nucleotide
SEQ ID NO:6 - amino acid 
ggctcgacgagcggcgggtcgacaagtggtggatctactagtggctctgga
G  S  T  S  G  G  S  T  S  G  G  S  T  S  G  S  G

gccacaatt
A  T  I
APT402 with optimized codons and bovine alpha-lactalbumin signal peptide SEQ ID NO:7 - nucleotide
SEQ ID NO:8 - amino acid 
atgatgtcctttgtctctctgctcctggttggcatcctattccatgccacccaggccgac
 M  M  S  F  V  S  L  L  L  V  G  I  L  F  H  A  T  Q  A  D
|alpha-lactalbumin signal peptide

gcgcccgaggtgctgcccccaggcctgaagtacgggatcgtcctggacgccggaagctcc
 A  P  E  V  L  P  P  G  L  K  Y  G  I  V  L  D  A  G  S  S
      |APT402 ->

ggtacccgggtctatgtgtaccagtggcccgccgaaaaggagaacaatacaggcgtggtg
 G  T  R  V  Y  V  Y  Q  W  P  A  E  K  E  N  N  T  G  V  V

tcacaaaccttcaagtgttcagtcaaaggtagtggaatttcttcatatggaaacaatccc
 S  Q  T  F  K  C  S  V  K  G  S  G  I  S  S  Y  G  N  N  P

caggatgtgcctagggcatttgaggaatgtatgcagaaagtcaagggccaggtcccatct
 Q  D  V  P  R  A  F  E  E  C  M  Q  K  V  K  G  Q  V  P  S

catctgcatggttcaactcccatccatctgggtgccactgcaggaatgaggctgctgcgg
 H  L  H  G  S  T  P  I  H  L  G  A  T  A  G  M  R  L  L  R

ctgcagaatgaaaccgcagccaacgaggtcctggaatccattcaaagttacttcaagtcc
 L  Q  N  E  T  A  A  N  E  V  L  E  S  I  Q  S  Y  F  K  S

caaccttttgacttccgaggcgctcaaattatcagtggtcaggaagagggagtctatggc
 Q  P  F  D  F  R  G  A  Q  I  I  S  G  Q  E  E  G  V  Y  G

tggatcaccgcaaactacctgatgggtaatttcctggagaagaacctgtggcatatgtgg
 W  I  T  A  N  Y  L  M  G  N  F  L  E  K  N  L  W  H  M  W

gtccatccccacggtgtcgaaacaactggagccctggatctgggcggtgccagtacacag
 V  H  P  H  G  V  E  T  T  G  A  L  D  L  G  G  A  S  T  Q
atctcatttgtcgctggtgagaaaatggacctgaacaccagcgatatcatgcaagtctct
 I  S  F  V  A  G  E  K  M  D  L  N  T  S  D  I  M  Q  V  S

ctgtatggatacgtctatactctgtacacccactctttccaatgttacggtcgcaacgaa
 L  Y  G  Y  V  Y  T  L  Y  T  H  S  F  Q  C  Y  G  R  N  E
 
gccgagaagaaattcctggctatgctgctgcaaaattctcctaccaagaatcatctgact
 A  E  K  K  F  L  A  M  L  L  Q  N  S  P  T  K  N  H  L  T

aatccttgctatcccagggactacagtattagctttactatgggccacgtgttcgattcc
 N  P  C  Y  P  R  D  Y  S  I  S  F  T  M  G  H  V  F  D  S

ctgtgcacagtcgaccagcggcccgagtcttacaatcccaacgatgtgatcaccttcgag
 L  C  T  V  D  Q  R  P  E  S  Y  N  P  N  D  V  I  T  F  E

ggcactggtgacccctcactgtgcaaggagaaagtcgcaagtatcttcgactttaaggct
 G  T  G  D  P  S  L  C  K  E  K  V  A  S  I  F  D  F  K  A

tgccacgatcaagaaacttgctcctttgatggcgtctatcaaccaaaaatcaagggccca
 C  H  D  Q  E  T  C  S  F  D  G  V  Y  Q  P  K  I  K  G  P

tttgtcgccttcgccgggttctattataccgcatccgccctgaacctgtctggtagcttc
 F  V  A  F  A  G  F  Y  Y  T  A  S  A  L  N  L  S  G  S  F

agtctggacacttttaattcaagtacatggaacttctgctctcagaattggagtcaactg
 S  L  D  T  F  N  S  S  T  W  N  F  C  S  Q  N  W  S  Q  L

cccctgctgctgcccaaattcgatgaggtgtacgctaggagttattgtttctcagcaaac
 P  L  L  L  P  K  F  D  E  V  Y  A  R  S  Y  C  F  S  A  N

tatatctaccatctgtttgtgaatggctacaaattcacagaggagacatggccacaaatt
 Y  I  Y  H  L  F  V  N  G  Y  K  F  T  E  E  T  W  P  Q  I

cactttgagaaagaggtcggaaattcatctattgcatggagcctggggtacatgctgagc
 H  F  E  K  E  V  G  N  S  S  I  A  W  S  L  G  Y  M  L  S

ctgactaatcagattcccgctgaatcacccctgatccgactgcctattgaaccacccgtc
 L  T  N  Q  I  P  A  E  S  P  L  I  R  L  P  I  E  P  P  V

ggctcgacgagcggcgggtcgacaagtggtggatctactagtggctctggagccacaatt
 G  S  T  S  G  G  S  T  S  G  G  S  T  S  G  S  G  A  T  I
|20 amino acid linker with S456A/G457T ->

gctcaagtgctgaggggaactgtgactgacttcccaggcttcgatgaaagggcagacgct
 A  Q  V  L  R  G  T  V  T  D  F  P  G  F  D  E  R  A  D  A
|Annexin V ->

gagactctgcgcaaagcaatgaagggtctgggtacagatgaagagagtattctgacactg
 E  T  L  R  K  A  M  K  G  L  G  T  D  E  E  S  I  L  T  L

ctgactagtcgctccaacgcccaaagacaggagattagcgcagcattcaaaactctgttc
 L  T  S  R  S  N  A  Q  R  Q  E  I  S  A  A  F  K  T  L  F

ggtagggatctgctggacgatctgaaatctgaactgaccgggaaattcgagaaactgatc
 G  R  D  L  L  D  D  L  K  S  E  L  T  G  K  F  E  K  L  I

gtggccctgatgaaaccaagtcggctgtatgatgcatacgaactgaagcacgctctgaag
 V  A  L  M  K  P  S  R  L  Y  D  A  Y  E  L  K  H  A  L  K
 
ggagccggcactaatgagaaggtgctgacagaaattatcgcatctcgaacccccgaggaa
 G  A  G  T  N  E  K  V  L  T  E  I  I  A  S  R  T  P  E  E

ctgcgcgctatcaagcaagtgtatgaagaggaatatggttccagcctggaggacgatgtc
 L  R  A  I  K  Q  V  Y  E  E  E  Y  G  S  S  L  E  D  D  V

gtcggggatacctcaggttattaccaacgcatgctggtggtgctgctgcaggccaacagg
 V  G  D  T  S  G  Y  Y  Q  R  M  L  V  V  L  L  Q  A  N  R

gaccctgacgctggcatcgatgaagcccaggtggaacaagacgcccaggcactgttccaa
 D  P  D  A  G  I  D  E  A  Q  V  E  Q  D  A  Q  A  L  F  Q

gcaggggagctgaagtgggggaccgacgaggaaaagtttatcaccatctttggaactcgg
 A  G  E  L  K  W  G  T  D  E  E  K  F  I  T  I  F  G  T  R

agtgtgagccatctgcgcaaagtctttgacaagtatatgacaatttccggcttccaaatc
 S  V  S  H  L  R  K  V  F  D  K  Y  M  T  I  S  G  F  Q  I

gaggaaaccattgatcgagagactagcggcaatctggaacagctgctgctggctgtggtg
 E  E  T  I  D  R  E  T  S  G  N  L  E  Q  L  L  L  A  V  V

aaatcaatcagatcaattcctgcatatctggcagagacactgtattacgcaatgaaagga
 K  S  I  R  S  I  P  A  Y  L  A  E  T  L  Y  Y  A  M  K  G

gccggaaccgacgatcatactctgattagagtcatggtcagtcggtccgagatcgacctg
 A  G  T  D  D  H  T  L  I  R  V  M  V  S  R  S  E  I  D  L

tttaacattagaaaggagttccgcaagaattttgcaacttccctgtactcaatgatcaaa
 F  N  I  R  K  E  F  R  K  N  F  A  T  S  L  Y  S  M  I  K

ggcgatacctctggtgactacaagaaagctctgctgctgctgtgcggcgaggatgactaa
 G  D  T  S  G  D  Y  K  K  A  L  L  L  L  C  G  E  D  D  -
APT402 with optimized codons
SEQ ID NO:9 - nucleotide
SEQ ID NO:10 - amino acid

gaggtgctgcccccaggcctgaagtacgggatcgtcctggacgccggaagctcc
E V L P P G L K Y G I V L D A G S S
|APT402 ->
ggtacccgggtctatgtgtaccagtggcccgccgaaaaggagaacaatacaggcgtggtg
 G T R V Y V Y Q W P A E K E N N T G V V

tcacaaaccttcaagtgttcagtcaaaggtagtggaatttcttcatatgggaacaatccc
 S Q T F K C S V K G S G I S S Y G N N P

caggatgtgcctagggcatttgaggaatgtatgcagaaagtcaagggccaggtcccatct
 Q D V P R A F E E C M Q K V K G Q V P S

catctgcatggttcaactcccatccatctgggtgccactgcaggaatgaggctgctgcgg

H L H G S T P I H L G A T A G M R L L R

ctgcagaatgaaaccgcagccaacgaggtcctggaatccattcaaagttacttcaagtcc
 L Q N E T A A N E V L E S I Q S Y F K S

caaccttttgacttccgaggcgctcaaattatcagtggtcaggaagagggagtctatggc
 Q P F D F R G A Q I I S G Q E E G V Y G

tggatcaccgcaaactacctgatgggtaattttctggagaagaacctgtggcatatgtgg
 W I T A N Y L M G N F L E K N L W H M W

gtccatccccacggtgtcgaaacaactggagccctggatctgggcggtgccagtacacag
 V H P H G V E T T G A L D L G G A S T Q
atctcatttgtcgctggtgagaaaatggacctgaacaccagcgatatcatgcaagtctct
 I S F V A G E K M D L N T S D I M Q V S

ctgtatggatacgtctatactctgtacacccactctttccaatgttacggtcgcaacgaa
 L Y G Y V Y T L Y T H S F Q C Y G R N E

gccgagaagaaattcctggctatgctgctgcaaaattctcctaccaagaatcatctgact
 A E K K F L A M L L Q N S P T K N H L T

aatccttgctatcccagggactacagtattagctttactatgggccacgtgttcgattcc
 N P C Y P R D Y S I S F T M G H V F D S

ctgtgcacagtcgaccagcggcccgagtcttacaatcccaacgatgtgatcaccttcgag
 L C T V D Q R P E S Y N P N D V I T F E

ggcactggtgacccctcactgtgcaaggagaaagtcgcaagtatcttcgactttaaggct
 G T G D P S L C K E K V A S I F D F K A

tgccacgatcaagaaacttgctcctttgatggcgtctatcaaccaaaaatcaagggccca
 C H D Q E T C S F D G V Y Q P K I K G P

tttgtcgccttcgccgggttctattataccgcatccgccctgaacctgtctggtagcttc
 F V A F A G F Y Y T A S A L N L S G S F

agtctggacacttttaattcaagtacatggaacttctgctctcagaattggagtcaactg
 S L D T F N S S T W N F C S Q N W S Q L

cccctgctgctgcccaaattcgatgaggtgtacgctaggagttattgtttctcagcaaac
 P L L L P K F D E V Y A R S Y C F S A N

tatatctaccatctgtttgtgaatggctacaaattcacagaggagacatggccacaaatt
 Y I Y H L F V N G Y K F T E E T W P Q I

cactttgagaaagaggtcggaaattcatctattgcatggagcctggggtacatgctgagc
 H F E K E V G N S S I A W S L G Y M L S

ctgactaatcagattcccgctgaatcacccctgatccgactgcctattgaaccacccgtc
 L T N Q I P A E S P L I R L P I E P P V

ggctcgacgagcggcgggtcgacaagtggtggatctactagtggctctggagccacaatt

 G  S  T  S  G  G  S  T  S  G  G  S  T  S  G  S  G  A  T  I
|20 amino acid linker with S456A/G457T ->

gctcaagtgctgaggggaactgtgactgacttcccaggcttcgatgaaagggcagacgct
 A  Q  V  L  R  G  T  V  T  D  F  P  G  F  D  E  R  A  D  A
|Annexin V ->

gagactctgcgcaaagcaatgaagggtctgggtacagatgaagagagtattctgacactg
 E  T  L  R  K  A  M  K  G  L  G  T  D  E  E  S  I  L  T  L

ctgactagtcgctccaacgcccaaagacaggagattagcgcagcattcaaaactctgttc
 L  T  S  R  S  N  A  Q  R  Q  E  I  S  A  A  F  K  T  L  F

ggtagggatctgctggacgatctgaaatctgaactgaccgggaaattcgagaaactgatc
 G  R  D  L  L  D  D  L  K  S  E  L  T  G  K  F  E  K  L  I

gtggccctgatgaaaccaagtcggctgtatgatgcatacgaactgaagcacgctctgaag
 V  A  L  M  K  P  S  R  L  Y  D  A  Y  E  L  K  H  A  L  K

ggagccggcactaatgagaaggtgctgacagaaattatcgcatctcgaacccccgaggaa
 G  A  G  T  N  E  K  V  L  T  E  I  I  A  S  R  T  P  E  E

ctgcgcgctatcaagcaagtgtatgaagaggaatatggttccagcctggaggacgatgtc
 L  R  A  I  K  Q  V  Y  E  E  E  Y  G  S  S  L  E  D  D  V

gtcggggatacctcaggttattaccaacgcatgctggtggtgctgctgcaggccaacagg
 V  G  D  T  S  G  Y  Y  Q  R  M  L  V  V  L  L  Q  A  N  R

gaccctgacgctggcatcgatgaagcccaggtggaacaagacgcccaggcactgttccaa
 D  P  D  A  G  I  D  E  A  Q  V  E  Q  D  A  Q  A  L  F  Q

gcaggggagctgaagtgggggaccgacgaggaaaagtttatcaccatctttggaactcgg
 A  G  E  L  K  W  G  T  D  E  E  K  F  I  T  I  F  G  T  R

agtgtgagccatctgcgcaaagtctttgacaagtatatgacaatttccggcttccaaatc
 S  V  S  H  L  R  K  V  F  D  K  Y  M  T  I  S  G  F  Q  I

gaggaaaccattgatcgagagactagcggcaatctggaacagctgctgctggctgtggtg
 E  E  T  I  D  R  E  T  S  G  N  L  E  Q  L  L  L  A  V  V

aaatcaatcagatcaattcctgcatatctggcagagacactgtattacgcaatgaaagga
 K  S  I  R  S  I  P  A  Y  L  A  E  T  L  Y  Y  A  M  K  G

gccggaaccgacgatcatactctgattagagtcatggtcagtcggtccgagatcgacctg
 A  G  T  D  D  H  T  L  I  R  V  M  V  S  R  S  E  I  D  L

tttaacattagaaaggagttccgcaagaattttgcaacttccctgtactcaatgatcaaa
 F  N  I  R  K  E  F  R  K  N  F  A  T  S  L  Y  S  M  I  K

ggcgatacctctggtgactacaagaaagctctgctgctgctgtgcggcgaggatgactaa
 G  D  T  S  G  D  Y  K  K  A  L  L  L  L  C  G  E  D  D  -

## Claims

1. A composition which comprises an apyrase and an annexin that binds phosphatidyl serine(PS) on the surface of activated platelets, said apyrase and annexin are present in amounts that are together effective to inhibit thrombosis without increasing bleeding.

2. The composition of claim 1 wherein the apyrase and annexin are present in equimolar amounts.

3. The composition of claim 2 wherein the apyrase and annexin are covalently coupled.

4. The composition of any of claims 1-3 wherein the apyrase is soluble CD39L3 or a modified form thereof with enhanced ADPase activity.

5. The composition of claim 4 wherein the apyrase is soluble CD39L3 in modified form wherein said modification consists of substitutions at amino acid positions 67 and 69; optionally wherein said substitutions are R67G and T69R, optionally wherein the apyrase is APT102 (SEQ ID NO:2).

6. The composition of any of claims 1-5 wherein the annexin is annexin IV or V.

7. The composition of any of claims 3-6 wherein the apyrase and annexin are covalently coupled through a linker peptide to form a fusion protein; optionally wherein the linker peptide contains 3-30 amino acids; optionally wherein the linker peptide contains 15-25 amino acids.

8. The composition of claim 7 wherein the linker peptide is resistant to hydrolysis in plasma and/or in cell culture supernatant.

9. The composition of claim 8 wherein the linker peptide has the amino acid sequence of SEQ ID NO:6.

10. The composition of claim 9 wherein the fusion protein is APT 402 (SEQ ID NO:10).

11. A nucleic acid that encodes the fusion protein as defined in any of claims 3-10.

12. Recombinant host cells or vectors that contain the nucleic acid of claim 11, wherein the nucleic acid further comprises control sequences for expression.

13. A method to produce a fusion protein that comprises apyrase and annexin covalently coupled through a linker peptide, which method comprises culturing the host cells of claim 12 and recovering the fusion protein from the culture.

14. A fusion protein that comprises apyrase and annexin covalently coupled through a linker peptide produced by the method of claim 13.

15. A composition of any of claims 1-10 for use in treating or preventing thrombosis.

## Patentansprüche

1. Zusammensetzung, die eine Apyrase und ein Annexin, das Phosphatidylserin (PS) an der Oberfläche aktivierter Thrombozyten bindet, enthält, wobei die Apyrase und das Annexin in Mengen vorhanden sind, die zusammen wirksam sind, Thrombosen zu hemmen, ohne Blutungen zu verstärken.

2. Zusammensetzung nach Anspruch 1, in der die Apyrase und das Annexin in äquimolaren Mengen vorhanden sind.

3. Zusammensetzung nach Anspruch 2, in der die Apyrase und das Annexin kovalent gekoppelt sind.

4. Zusammensetzung nach einem der Ansprüche 1-3, in der es sich bei der Apyrase um lösliches CD39L3 oder eine modifizierte Form davon mit erhöhter ADPase-Aktivität handelt.

5. Zusammensetzung nach Anspruch 4, in der es sich bei der Apyrase um lösliches CD39L3 in modifizierter Form handelt, in der die besagte Modifikation aus Substitutionen an den Aminosäurepositionen 67 und 69 besteht; gegebenenfalls in der es sei bei den besagten Substitutionen um R67G und T69R handelt, gegebenenfalls in der es sich bei der Apyrase um APT102 (SEQ ID NO: 2) handelt.

6. Zusammensetzung nach einem der Ansprüche 1-5, in der es sich bei dem Annexin um Annexin IV oder V handelt.

7. Zusammensetzung nach einem der Ansprüche 3 bis 6, in der die Apyrase und das Annexin durch ein Linkerpeptid kovalent gekoppelt sind, um ein Fusionsprotein zu bilden; gegebenenfalls in der das Linkerpeptid 3 bis 30 Aminosäuren enthält; gegebenenfalls in der das Linkerpeptid 15 bis 25 Aminosäuren enthält.

8. Zusammensetzung nach Anspruch 7, in der das Linker-Peptid gegen Hydrolyse in Plasma und/oder in Zellkulturüberstand resistent ist.

9. Zusammensetzung nach Anspruch 8, in der das Linker-Peptid die Aminosäuresequenz von SEQ ID NO:6 aufweist.

10. Zusammensetzung nach Anspruch 9, in der es sich beim Fusionsprotein um APT 402 (SEQ ID NO: 10) handelt.

11. Nukleinsäure, die das Fusionsprotein nach einem der Ansprüche 3-10 kodiert.

12. Rekombinante Wirtszellen oder Vektoren, die die Nukleinsäure nach Anspruch 11 enthalten, wobei die Nukleinsäure ferner Kontrollsequenzen zur Expression umfasst.

13. Verfahren zur Herstellung eines Fusionsproteins, das Apyrase und Annexin enthält, die durch ein Linker-Peptid kovalent gekoppelt sind, wobei das Verfahren die Kultivierung der Wirtszellen nach Anspruch 12 und die Gewinnung des Fusionsproteins aus der Kultur umfasst.

14. Fusionsprotein, das Apyrase und Annexin enthält, die kovalent durch ein Linkerpeptid gekoppelt sind, hergestellt nach dem Verfahren gemäß Anspruch 13.

15. Zusammensetzung nach einem der Ansprüche 1-10 zur Verwendung bei der Behandlung oder Vorbeugung von Thrombose.

## Revendications

1. Composition comprenant une apyrase et une annexine liant la phosphatidylsérine (PS) à la surface de plaquettes activées, lesdites apyrase et annexine étant présentes en des quantités conjointement efficaces pour inhiber la thrombose sans augmenter le risque de saignement.

2. Composition selon la revendication 1, l'apyrase et l'annexine étant présentes en quantités équimolaires.

3. Composition selon la revendication 2, l'apyrase et l'annexine étant liées de façon covalente.

4. Composition selon l'une quelconque des revendications 1 à 3, l'apyrase étant un CD39L3 soluble ou une forme modifiée de celui-ci présentant une activité ADPase accrue.

5. Composition selon la revendication 4, l'apyrase étant un CD39L3 soluble sous forme modifiée, ladite modification consistant en des substitutions aux positions d'acides aminés 67 et 69, lesdites substitutions étant éventuellement R67G et T69R, ladite apyrase étant éventuellement APT102 (SEQ ID NO: 2).

6. Composition selon l'une quelconque des revendications 1 à 5, l'annexine étant l'annexine IV ou V.

7. Composition selon l'une quelconque des revendications 3 à 6, l'apyrase et l'annexine étant liées de façon covalente par l'intermédiaire d'un peptide de liaison pour former une protéine de fusion, ledit peptide de liaison contenant éventuellement 3 à 30 ou 15 à 25 acides aminés.

8. Composition selon la revendication 7, le peptide de liaison étant résistant à l'hydrolyse dans le plasma ou dans le surnageant de culture cellulaire.

9. Composition selon la revendication 8, le peptide de liaison ayant la séquence d'acides aminés SEQ ID NO:6.

10. Composition selon la revendication 9, la protéine de fusion étant APT 402 (SEQ ID NO : 10).

11. Acide nucléique codant pour la protéine de fusion telle que définie dans l'une quelconque des revendications 3 à 10.

12. Cellules hôtes recombinantes ou vecteurs recombinants contenant un acide nucléique selon la revendication 11, ledit acide nucléique comprenant en outre des séquences régulatrices de l'expression.

13. Procédé permettant de produire une protéine de fusion comprenant une apyrase et une annexine liées de façon covalente par l'intermédiaire d'un peptide de liaison, ledit procédé comprenant la culture des cellules hôtes selon la revendication 12 et la récupération de la protéine de fusion à partir de ladite culture.

14. Protéine de fusion comprenant une apyrase et une annexine liées de façon covalente par l'intermédiaire d'un peptide de liaison produit par le procédé selon la revendication 13.

15. Composition selon l'une quelconque des revendications 1 à 10, destinée à être utilisée dans le traitement ou la prévention de la thrombose.
